# EUROPEAN PATENT APPLICATION

(11) **EP 2 008 661 A2**
(43) Date of publication of application: **31.12.2008**
(21) Application number: 08380137.3
(22) Date of filing: 05.05.2008
(51) Int. Cl.: A61K 36/886, A61K 36/28

(54) **Formulation based on marigold aloe and centellae**

(30) Priority: 03.05.2007 AR P070101909
(71) Applicant: Spannagel, Lucia Antonia, Guemes 235, barrio Los Eucaliptos Villa Carlos Paz Provincia de Cordoba (AR)
(72) Inventor: Spannagel, Lucia Antonia, Guemes 235, barrio Los Eucaliptos Villa Carlos Paz Provincia de Cordoba (AR)
(74) Representative: Fernandez Lerroux, Aurelio

(57) **Abstract**

The formulation includes between 1 and 98 partsof glycolic extract of marigold officinalis, between1 and 98 parts of glycolic extract of aloe vera and between 1 and 98 parts of glycolic extract of centellae asiaticae.

The formulation conforms an emulsion from the use of between4 and 8 % of glycolic extract of marigold officinalis, between 4 and 8 % of glycolic extract of aloe vera and between 5,5 and 9 % of glycolic extract of centellae asiaticae incorporated into a base cream non ionic that includes an oily phase where it is included between 6,5 and 12 % of selfemulsive wax non ionic; between 3,2 and 5,8 % of estearic acid of triple pressure; between 4 and 6,4 % of solid Vaseline(petroleum jelly) and between7,6 and 9,7 % of liquid Vaseline (petroleum jelly).

In turn, the watery phase includes between 7 and 10,4 % of glycerine; between 0,1 and 0,8 % of imidozolindil urea; between a 1,6 and 3,3 % of preserving universal; between 0,7 and 1,5 % of polyethilenglycol 400; between 0,35 and 0,78 % of Twen 80.-

Finally the emulsion includes the addition of demineralized water up to completing 100 g. of product.

## Description

The present invention consists of a formulation based on marigold, aloe and centellae.

In order to make the present invention understandable so that it can be easily taken to practice, it will be given in the paragraphs that follow a precise description of a favorite form of accomplishment, and which formulation is exemplified incorporated into a basic form by a watery phase and an oily phase by purely demonstrative but not limitative character of the invention, which components will be able to combine with such diverse compounds as those who are exemplified in the report without it implying separating of the principles of the invention established in the present documentation.-

OBJECT - PREVIOUS ART: innumerable compounds exist in the previous art with anti-inflammatory, antiseptic and healing properties. Also in the previous art there are well known the use of the compounds of the emulsion that reveals. It is possible to say in general that such properties have been investigated necessarily from the times of our first ancestors in order to solve the continuous problems that were presented.

From observation, men have been progressing in the discovery of the properties of the beings that surround us and from the ingenuity such properties have sought to take advantage in their own benefit.-

The plants and animals, from the need of food, have been the objects of the first observations, and later, those who have allowed to relate the reactions of the animals exposed to certain types of plants.

This way is how could have determined the benign or harmful factors present in the plants.

The first ones were looked when the animal needed to restore its health, the second ones were looked by some species as defense from predators or avoided for them.

Such there is at the time the origin of the medicines and poisons.

Along History, we have studied numerous species of plants and still when we have not studied all of them, partly because many remain ignored and partly because many others have disappeared already, and we have achieved innumerable medicines derived from its leaves, stems, flowers and roots.

Due to the fact that many of such species are known from long time ago, we rely on an extensive bibliography related to the studies, tests, results and analysis as turns out to be the case of the elements that are in use in the formulation which protection is solicited.

The marigold is an annual plant of the family of composed widely distributed all over the world being their medicinal properties and colourings well known from old times, especially for the Arabians and Indians and then for the Greek.

We have notes on the applications recommended by the Abbess Hildegarda of Bingen (1099-1179) in the treatment of the impetigo and the dermal stains and it was considered of high therapeutic value by Albertus Magnus (1193-1280) being incorporated in almost all the herbal texts known about that time.

Closer in history we see that the father Sebastian Kneipp (1821-1897) was recommending the marigold for the treatment of sores, varicose veins and dermatological spots diseases.

Diverse salves prepared on the basis of marigold have been applied like anti-inflammatory and antiseptic in the North American Civil War as well as in the First World War.

The aloe is another of the principal ingredients of the formulation which protection is being solicited. The aloe is a plant of the family of the liliaceous that also was recognized by its antiseptic and healing power from long data.

There are known approximately 700 varieties of this plant that nowadays we use in the elaboration of so diverse products as food, paintings and cosmetics. -Among the oldest quotations that we find, the reference to the Bible is mandatory where it is reported how, after bringing down the cross the body of our Lord, was anointed it with a salve prepared with myrrh and aloe.

Also it appears in Dioscorides's herbalist's of the 1 st century dC that Alexander the Great conquered the island Socotorra, South of Arabia, because there was finding of a great quantity of aloe, which would serve for the healing of wounds and diseases of his soldiers during the conquers. It is known that the Chinese were the first ones in using the aloe, and in the Old Egypt also it was of frequent use.

For Ricardo Gampel, immune-pharmacologist and specialist in Natural Therapies of the University of Buenos Aires, the utilization of the aloe goes together with the development of the humanity from its origins since is documented among the Chinese, Indians, Sumerian, Assyrians, Babylonians, Egyptians and Hebrews.

The most common uses were given in the treatment of wounds, injuries, burns, affections of the skin, indigestion and gases.

In the already mentioned herbalist, Dioscorides does a wide description of the plant for its medicinal and cosmetic properties. - Introduced in the current Málaga for the Arabians, big extensions were cultivated until the modern pharmacopoeia relegated the aloe together with many other medicinal plants.

The rediscovery of this plant took place during the Second World War, when it was used in the treatment of the radioactive burns of the people from Hiroshima and Nagasaki.

The references indicate that the injuries recovered more quickly and in many cases without leaving signs or scars.

More than 60 % of the solids of the total of the aloe are polysaccharides mucilaginous ligatures to sugars as glucose, manose, ramnose, xilosa, arabinosa, galactosa and acids urónicos. The slime is composed of different neutral, acid polysaccharides and acetilados such as mananos, glucomananos, galactomananos, etc., which are in charge of the great capacity that has the plant to retain water, as well as the aloerido that is a polysaccharide of high molecular weight.

The remaining solid ones are organic salts and acids as the glutámico, málico, salicílico, citric, lactato magnesium, oxalato calcic, etc.; such enzymes as the celulasa, carboxipeptidasa, bradikininasa, catalasa, amylase, oxidasa and tirosinasa; sapogenics; tannins; esteroles; triglicerids; amino acids as the lisina, histidina, glutamina, arginina, aspartic acid, asparagina, treonina, serina, glutamic acid, glycine, alanina, valina, metionina, isoleucine, leucine, tirosine, fenilalanine and triptofano; RNA and traces of alkaloids; betacarotens; vitamins B1, B2, B3, B6, C, And; colina; folic acid and minerals like aluminium, boron, barium, calcium, chrome, copper, iron, potassium, magnesium, sodium, phosphorus, strontium and silicon.

It can be said that the aloe is indicated for internal and external use in dermatological affections; exanthematic infections as measles, chicken pox, rubella and herpes; affections of mucous gastric and intestinal; gastrointestinal infections and inflammatory intestinal diseases as crohn, ulcerous colitis and irritable colon and of the buccal mucous aphthas, gingivitis, periodontitis, candidiasis mouth and esofagica), conditions(states) of immunosuppretion, inflammatory and autoimmune processes type arthritis, tumour processes, prevention of conditions(states) of immunosuppretion and infectious processes, hyperglycaemia and hyperlipidaemia.

Adapted for its use in processes of dermal cicatrization, the centellae regulates quantitatively and qualitatively the new conjunctive tissue allowing to obtain a soft, flexible tissue and not hypertrophic one.

As an extract the centellae regulates the metabolism of the conjunctive tissue stimulating the synthesis of collagen and of the glycosaminoglycans.

It acts in the fibroblast of the conjunctive tissue intervening in the enzymatical activity that controls the fixation of prolina and alanina to the ARN matricials that will take part in the biosynthesis of collagen forming collagen I and collagen III as well as the normal skin and vasculars walls.

It regulates the proliferation of the fibroblasts leading them to the obtaining, in quantity and quality of the required collagen, avoiding this way the formation of keloids and orientates them towards the preferential synthesis of collagen. Is through the fibroblasts that controls the production of the collagenous fibers, when the process of cellular regeneration is disturbed or desorganized.

The centellae was known from ancient times and its leaves were used in Madagascar and India for the treatment of wounds, sores, ulcers and leprosy in the form of fesh juice of the plant.

In its composition the content emphasizes in saponins triterpenics, sugars, essential oils and tanins.

It possesses a great healing power on the basis of the stimulation of the cellular mitosis favoring the biosynthesis of the collagen to level the conjunctive tissues.

Among the elements that can intervene in obtaining the results we can find the aminados and abundant and varied mineral salts.

The centellae is a very resistant climbing plant that grows in the tropics. Also known as " herb of the tiger "because the hunters were following the track of these beasts and observing these plants of centellae, since the wounded animals had the habit of rolling about in the shrubs to recover.

Doing a research to determine the documents that could be considered to be precedents of this proposed invention, there have been located the patents CN1176134; CN1256128; CN1288741; CN1302654; CN1302661; CN1313106; CN1359701; CN1366992; CN1404858; CN1559580; CN1679643; DE10242138; DE19844255; JP2117618; JP5112458; JP57145806; RU2128500; US2005025723; US4725438; W002069884 and W02004039390 all which say to compositions that they include aloe or some derivative of aloe but none of the remaining components that are revealed in this file.

Likewise such patents of invention have been located as the US5266330; US5843467 and US5997876 documents in which the utilization of marigold is revealed, but as in case of the documents referred to the aloe, the same one is in use without the addition of any of the remaining components of the invention that is being revealed.

Finally there also have been located the documents RU2138277 and RU2149009 that claim products and procedures where the above mentioned products have been elaborated on the basis of plants and herbs that do not guard relation with the developing in the present documentation.

DESCRIPTION: Basically the present invention consists of a formulation based on marigold, aloe and centellae obtained from glycolic extracts of flowers of marigold, air parts of centellae and gel of aloe that can be used as powder or talc, can join to a base and can include other components.

In a favorite form of accomplishment the formulation that is revealed is used as an emulsion incorporating it into a base that presents an oily phase and a watery phase.

FUNCTIONING: Once established the diverse components that explain the nature of the invention, it has to be complemented immediately afterwards with the functional and operative relation of the same as well as the result that they provide.

In order to possess a product for the cure of diverse affections of the skin that favors the granulation and the later cicatrization, turning out to be a natural, non aggressive and humid product that proceeds to accomplishes a formulation based on Marigold, Aloe and Centellae.

The first aspect that must be highlighted is that there is not known a formulation in which these three components appear together.

From the research carried out and according to the documents founded as well as the consulted bibliography, it arises that each of the components has been studied for a long time across the history and for such motive their properties have been taken advantage under diverse preparations.

Nevertheless the inventor is the one that, from her investigation and experimentation, has brought them together for the first time in the formulation that is being revealed.

In the document is specified a formula in which three components are incorporated to a base cream, and in the same one there are given the proportions for them.

Nevertheless, according to the experience of the authoress, the three mentioned components can be incorporated in any proportion since the major or minor quantity of one of them respect of other two it allows to obtain, not only different properties but also different physical characteristics with what the resultant formula of the different combinations allows to obtain a compound that will turn out to be more adapted for the treatment of a certain ailment, or it will give a consistency that will favor its use as lotion.

Among the characteristics of the product that is revealed it has the capacity of acting from the internal layers towards the exterior, provoking the drainage of the wound, for what if there was infection this one will not stay inside while the superficial layers are healing.

The formulation includes then between 1 and 98 parts of a glycolic extract of marigold to which are added between 1 and 98 parts of a glycolic extract of aloe and between 1 and 98 parts of a glycolic extract of centellae.

A variant of the above mentioned formulation includes that, per every part of glycolic extract of marigold are added between 1 and 2 parts of a glycolic extract of aloe and between 1,3 and 2,4 parts of a glycolic extract of centellae.

The formulation which needs to be protected can be used as a powder or talc, or can be incorporated into a base for its utilization as a paste, a solution, a lotion, a gel, a cream or an ointment.

The proposed formulation allows to be combined by other elements and chemical products such as exicipients, antibiotics, vitamins and corticoids for example.

The preferred way of using the formulation that is being revealed consists of making an emulsion incorporating into a base cream non ionic that has an oily phase and a watery phase.

In this form of emulsion is conformed from the incorporation of between 4 and 8 % of glycolic extract of marigold, between 4 and 8 % of glycolic extract of aloe and between 5,5 and 9 % of glycolic extract from centellae to a base cream non ionic in which the oily phase includes between 6,5 and 12 % of selfemulsive wax non ionic; between 3,2 and 5,8 % of estearic acid of triple pressure; between 4 and 6,4 % of solid Vaseline (petroleum jelly) and between 7,6 and 9,7 % of liquid Vaseline (petroleum jelly).

The watery phase of the above mentioned emulsion includes between 7 and 10,4 % of glycerine; between 0,1 and 0,8 % of imidozolindil urea; between a 1,6 and 3,3 % of preserving universal; between 0,7 and 1,5 % of polyethilenglycol 400; between 0,35 and 0,78 % of Twen 80 and demineralized water up to completing the total.

In the present documentation one refers to the marigold, to the aloe and to the centellae, must be understood that such a reference is done bearing in mind that they are respectively the marigold officinalis, the aloe vera and the centellae asiaticae.

Also it has to be understood that in the proposed formula the percentage of each one of the active principles, must be understood like recounted to a relation volume/weight.

Finally, itcorresponds to demonstrate that the glycolic extract of marigold is a transparent liquid of golden and soluble tone in water that has been obtained from the flowers of the plant.

In relation to the glycolic extract of the aloe it consists of a transparent liquid of beige-golden soluble color of water and obtained from the gel of the plant.

Preferably, the above mentioned gel has been obtained by a procedure of stabilization that it turns out to be acquainted in the previous art.

In relation with the glycolic extract of centellae, it consists of a transparent liquid of golden tone that turns out to be soluble in water to a temperature of 25 ° C (77 degrees F) and is obtained of the air parts of the plant.

The glycolic extracts of marigold, aloe and centellae used in the formulation that is revealed, have a density of between 1,0 and 1,1 and 1,0 and 1,2 grams / milliliter and the pH of the same ones is considered between 4 and 7.

This way it is been outlined one of the constructive possibilities that lead to concrete the invention and also the way it works, and implying its specific applications. The documentation is complemented with the synthesis of the invention contained in the following claims.

Having done the descriptions and determined the nature of the invention, its possibilities and the it can be taken practice in its fundaments is declared as invention and of exclusive property as it follows:

## Claims

1. **Formulation based on marigold, aloe and centellae characterized** because it includes between 1 and 98 parts of a glycolic extract of marigold officinalis, between 1 and 98 parts of a glycolic extract of aloe vera and between 1 and 98 parts of a glycolic extract of centellae asiaticae.

2. Formulation, conformity with the claim number 1, **characterized** because for every part of glycolic extract of marigold officinalis, is added between 1 and 2 partsof extract of aloe vera and between 1,3 and 2,4 parts of glycolic extract of centellae asiaticae.

3. Formulation, conformity with the claim number 1, **characterized** because it consists of an emulsion conformed by incorporation of between 4 and 8 % of glycolic extract of marigold officinalis, between 4 and 8 % of glycolic extract of aloe vera and between 5,5 and 9 % of glycolic extract of centellae asiaticae to a base cream non ionic that includes an oily phase where have been added between 6,5 and 12 % of selfemulsive wax non ionic; between 3,2 and 5,8 % of estearic acid of triple pressure; between 4 and 6,4 % of solid Vaseline (petroleum jelly) and between 7,6 and 9,7 % of liquid Vaseline(petroleum jelly) and also including a watery phase where are incorporated between 7 and 10,4 % of glycerine; between 0,1 and 0,8 % of imidozolindil urea; between a 1,6 and 3,3 % of preserving universal; between 0,7 and 1,5 % of polyethilenglycol 400; between 0,35 and 0,78 % of Twen 80 and demineralized water up to completing 100 g. of product; and which products that are included in the formulation are recounted to a percentage measured in relation to volume / weight.

4. Formulation, conformity with the claim number 1, **characterized** because the glycolic extract of marigold, the glycolic extract of aloe vera and the glycolic extract of centellae used in this formulation, are obtained respectively of the flowers of the marigold, of a gel of the aloe and of the air parts of the centellae.

5. Formulation, conformity with the claim number 1, **characterized** because the glycolic extracts of marigold, aloe and centellae used in the formulation, have a density that includes between among 1,0 - 1,1 and 1,0 - 1,2 grams / milliliter.

6. Formulation, conformity with the claim number 1, **characterized** because the pH of the glycolic extracts of marigold, aloe and centellae includes between 4 and 7.-

7. Formulation, conformity with the claim number 1, **characterized** because the gel of the aloe is obtained by estabilization.

8. Formulation, conformity with the claim number 1, **characterized** because the formulation can be used as a powder or talc.

9. Formulation, conformity with the claim number 1, **characterized** because it can be incorporated to a base for its utilization as a paste, a solution, a lotion, a cream, a gel or an ointment.

10. Formulation, conformity with the claim number 1, **characterized** because it can be combined by at least one element selected among exicipients, antibiotics, corticoids, vitamins and a chemical product.
